# EUROPÄISCHE PATENTSCHRIFT

(11) **EP 2 246 699 B1**
(45) Veröffentlichungstag und Bekanntmachung des Hinweises auf die Patenterteilung: **20.06.2012**
(21) Anmeldenummer: 10004427.0
(22) Anmeldetag: 27.04.2010
(51) Int. Cl.: G01N 33/50

(54) **Verfahren zum Selektieren, Screening, von Blut und/oder Serum auf granulozytäre Antikörper bei umfangreicher Spenderpopulation**
Method for selecting and screening blood and/or serum for granulocytic antibodies in an extensive donor population
Procédé pour sélectionner et cribler du sang et/ou du sérum pour des anticorps granulocytaires dans une vaste population de donneurs

(30) Priorität: 27.04.2009 DE 102009019013
(43) Veröffentlichungstag der Anmeldung: 03.11.2010
(73) Patentinhaber: DRK Blutspendedienst Baden-Würtemberg-Hessen GmbH, 76530 Baden-Baden (DE)
(72) Erfinder: Nguyen, Xuan Duc Dr., 68309 Mannheim (DE)
(74) Vertreter: Vonnemann, Gerhard

(56) Entgegenhaltungen:
- EP-A2- 2 006 375
- STRONCEK ET AL: "Leukocyte Antigen and Antibody Detection Assays: Tools for Assessing and Preventing Pulmonary Transfusion Reactions" TRANSFUSION MEDICINE REVIEWS, GRUNE AND STRATTON, ORLANDO, FL, US LNKD- DOI:10.1016/J.TMRV.2007.05.003, Bd. 21, Nr. 4, 24. September 2007 (2007-09-24), Seiten 1-20, XP002590851 ISSN: 0887-7963
- PORRETTI LAURA ET AL: "Flow-cytometric approach to the prompt laboratory diagnosis of TRALI: a case report" EUROPEAN JOURNAL OF HAEMATOLOGY, Bd. 73, Nr. 4, Oktober 2004 (2004-10), Seiten 295-299, XP002588911 ISSN: 0902-4441
- STRONCEK D: "Granulocyte antigens and antibody detection" Juli 2004 (2004-07), VOX SANGUINIS, SUPPLEMENT 200407 GB, VOL. 87, NR. 1, PAGE(S) S91-S94 , XP002588912 ISSN: 1741-6892 * das ganze Dokument *
- DAVOREN A ET AL: "TRALI due to granulocyte-agglutinating human neutrophil antigen-3a (5b) alloantibodies in donor plasma: a report of 2 fatalities" TRANSFUSION, AMERICAN ASSOCIATION OF BLOOD BANKS, BETHESDA, MD, US, Bd. 43, Nr. 5, 1. Mai 2003 (2003-05-01), Seiten 641-645, XP009125847 ISSN: 0041-1132

## Beschreibung

### Technisches Gebiet.

Die Erfindung betrifft ein Verfahren zum Selektieren, Screening, von Blut und/oder Serum auf granulozytäre Antikörper bei umfangreicher Spenderpopulation.

### Stand der Technik:

Es ist bekannt, dass Leukocyten-assoziierte Antikörper zu ernsten Transfusionreaktionen bei Blut- oder Plasmaspendeempfängern, insbesondere von deren Lunge (TRALI; siehe: 1. Popovsky MA, Chaplin HC Jr, Moore SB. Transfusion-related acute lung injury: a neglected, serious complication of hemotherapy. Transfusion 1992; 32: 589-592; 2. Silliman CC. Transfusion-related acute lung injury. Transfus Med Rev 1999; 13: 177-186), oder zu flebrigen Transfusionsreaktionen, bei der Verabreichung von Blut- oder Plasmaspenden führen können, insbesondere, wenn granulozytär-spezifische HNA-3a-Antikörper in der Spende vorhanden sind. Groß angelegte Analyseuntersuchungen zeigen die Bedeutung der granulozytäre Antikörper auf, so dass deren Vorhandensein oder deren Abwesenheit in Spenderblut wie in Spender plasmaprodukten bekannt sein sollte, um die Verabreichung von Blutspenden und/oder Plasma mit granulozytären Antikörpern nach Möglichkeit auszuschließen. Granulozyten, Mikrophagen, sind polymorphkemige Leukozyten, welche wohl differenziert, jedoch nicht mehr teilungsfähig sind und ein granuliertes Zytoplasma besitzen.

Die Pathogenese von TRALI schließt die Infusion von spezifischen Antikörpern des Spenders ein, gerichtet gegen Antigene, nämlich HLA Klasse I oder Klasse II, oder granulozytär-spezifisch, welche auf den Leukozyten des Empfängers vorhanden sein können, als Resultat von Komplement-Aktivierungen, von neutrophilen (PMN) Sequestrationen sowie Aktivierungen in der Lunge, was zur endothelialen Zerstörung und Kapillarausfällen sowie akuten Lungenverletzungen (ALI) führen kann. Antikörper HLA Klasse II und HNA, insbesondere HNA 3a, sind in den meisten klinischen Fällen von TRALI betroffen.

Als Verfahren der Granulozytendiagnostik zum Screening von Blut und/oder Plasma zur Detektion von granulozytären Antikörpern ist der Granulozyten-Agglutinations-Test am Mikroskop, abgekürzt GAT genannt, sowie der Granulozyten-Immunfluoreszenz-Test am Mikroskop, abgekürzt GIFT genannt, bekannt (siehe: 1. Lalezari P, Jiang A, Lee S: A microagglutinination technique for the detection of leucocyte agglutinatins; in Ray JG (ed.): NIAID Manual of Tissue Typing Techniques, NIH Publication. Rockville, National Institutes of Health, 1979:20-2; 2. Verheugt FW, von dem Borne AE, Décary F, Engelfriet CP The detection of granulocyte alloantibodies with an indirect immunofluorescence test. .Br J Haematol. 1977;36;533-44). Fällt der Granulozyten-Agglutinationstest (GAT) oder der Granulozyten-Immunfluoreszenztest (GIFT) positive aus, so wird zur Spezifizierung der nachgewiesenen Antikörper MAIGA angewendet.

Bei den bekannten Verfahren wird folgendermaßen vorgegangen: Es wird im Prinzip EDTA-Blut von wenigstens 3 HNA-typisierten Spendern untersucht, indem eine Zellisolierung über den Dichtegradient erfolgt. Anschließend werden die Proben gewaschen und es erfolgt eines Lyse von Resterythrozyten, wonach nochmals eine Waschung durchgeführt wird. Nach diesem Waschschritt teilen sich die Verfahren GIFT und GAT auf.

Hinsichtlich des Granulozyten-Immunfluoreszenz-Tests am Mikroskop erfolgt eine Fixierung mit PFA sowie ein Waschvorgang. Anschließend erfolgt eine Inkubation mit Serum sowie eine Inkubation mit Fluoreszenz-markierten Antikörpern. Das Ergebnis wird einer mikroskopischen Analyse unterzogen. Hinsichtlich des Granulozyten-Agglutinations-Tests am Mikroskop erfolgt eine Inkubation mit Serum, wonach dieses Ergebnis einer mikroskopischen Analyse unterzogen wird. Die Nachteile der bisherigen Methoden bestehen darin, dass diese sehr zeitaufwendig sind und 5 bis 6 Stunden bis zum Abschluss der mikroskopischen Analyse benötigen. Des Weiteren ist nur ein geringer Durchsatz möglich, mit welchem ein hoher Verbrauch an Testzellen durch hohen Zellverlust bei der Zellisolierung einhergeht. Ein Screening von Blut bzw. Serum umfangreicher Spenderpopulation ist mit den bekannten Verfahren GIFT und GAT nicht möglich.

Des weiteren ist eine Durchflusszytometrie als sensitive Methode für die Detektion von granulozytär-gebundenen Immunoglobulinen bekannt geworden (Veys PA, Gutteridge CN, Macey M, Ord J et al. Detection of granulocyte antibodies using flow cytometric analysis of leucocyte immunofluorescence. Vox Sang 1989;56:42-7). Jedoch erfordert diese Methode die Isolation der Granulozyten, wobei ein hoher Verlust von Zellen in Kauf genommen werden muss.

Im Stand der Technik ist es deshalb notwendig, die Standart GIFT- und GAT-Verfahren anzuwenden, um alle relevanten Antikörpern HNA, insbesondere HNA-3a Antikörper zu detektieren, welche am besten durch das GAT-Verfahren detektiert werden können (siehe: 1. Bux J, Chapman J: Results of the Second International Granulocyte Serology Workshop. Transfusion 1997;37:977-83; 2. Lucas G, Rogers S, de Haas M et al. Report on the Fourth International Granulocyte Immunology Workshop: progress toward quality assessment. Transfusion. 2002 Apr;42:462-8).

### Technische Aufgabe:

Der Erfindung liegt die Aufgabe zugrunde, ein Verfahren zum Selektieren, Screening, von Blut und/oder Plasma auf granulozytäre Antikörper zu schaffen, um zahlreiche Blutproben umfangreicher Spenderpopulation rasch untersuchen zu können, insbesondere in einem automatisierten Verfahren; ebenso soll für das Verfahren nur ein geringer Verbrauch von Testzellen notwendig sein. Insbesondere soll das Verfahren es ermöglichen, dass solche granulozytären Antikörper aufgefunden werden können, welche bisher nur in der Kombination von GIFT-und GAT-Verfahren detektiert werden können.

Die Lösung der Aufgabe besteht patentgemäß darin, dass zum Selektieren, Screening, von Blut und/oder Serum auf granulozytäre Antikörper bei umfangreicher Spenderpopulation eine Durchflusszytometrie mit Granulozyten-Immunfluoreszenz angewendet wird, wobei EDTA-Blut von 2 -HNA-typisierten Spendern verwendet wird und eine Zellisolierung über den Dichtegradient mittels Vollblutlyse erfolgt unter Ausschluss einer Zentrifugation und anschließend ein Waschschritt erfolgt, wonach zur Durchführung des Granulozyten-Immunfluoreszenz-Tests eine Inkubation des isolierten Zellmaterials mittels Serum sowie eine Inkubation mit fluoreszenz-markierten Antikörpern erfolgt und das inkubierte Zellmaterial einer Durchfluss-zytometrischen Analyse unterworfen wird.

In weiterer erfinderischer Ausgestaltung erfolgt diese Durchfluss-zytometrische Analyse in einem automatisierten Verfahren.

Der Vorteil des erfindungsgemäßen Verfahrens zum Selektierten, Screening, von Blut und/oder Plasma auf granulozytäre Antikörper besteht insbesondere darin, dass es eine Durchfluss-zytometrische Analyse zur Verfügung stellt, mit welcher zahlreiche Blutproben umfangreicher Spenderpopulationen untersucht werden können, insbesondere in einem automatisierten Verfahren. Durch die Vermeidung eines Schrittes der Zentrifugation ist nur ein geringer Verbrauch an Testzellen gegeben, weit insbesondere die Zentrifugation zur Zerstörung und Unbrauchbarkeit der Testzellen führt. Ein weiterer Vorteil des erfindungsgemäßen Verfahrens besteht darin, dass nunmehr auch solche granulozytären Antikörper aufgefunden werden, welche bisher gemäß des Standes der Technik nur in der Kombination von GIFT- und GAT-Verfahren detektiert werden konnten. Ein weiterer Vorteil besteht darin, dass das erfindungsgemäße Verfahren in rascher Abfolge eine Vielzahl von Untersuchungen durchzuführen gestattet. Beispielsweise werden für 50 Untersuchungen nur drei bis höchstens vier Stunden benötigt, wohingegen beim Stand der Technik für einige wenige Untersuchungen fünf bis 6 Stunden benötigt werden.

In weiterer erfindungsgemäßer Ausgestaltung wird jede Zellpopulation bei der Durchführung des Ficoll-Dichtegradienten zur Zellisolierung durch Sedimentation getrennt, wodurch Granulozyten, Monozyten und Lymphozyten in ausreichender Anzahl für den Test einer großen Zahl von Blutproben erhalten werden.

Weitere vorteilhafte Ausgestaltungen des Verfahrens sind in den Unteransprüchen beschrieben.

Kurzbeschreibung der Zeichnung, in der zeigen:
- Figur 1: Protokoll zur Analyse von Zell-bindender Fluoreszenz auf Granulozyten (GRC), Monozyten (MO) und Lymphozyten (LY)
- Figur 2: ein Beispiel einer durchflusszytometrischen Analyse eines Serums mit Granulozyten-spezifischem IgG Anti-HNA-3a und
- Figur 3: ein Beispiel einer durchflusszytometrischen Analyse eines Serums mit zytotoxischen und nicht-zytotoxischen Antikörpern gegen HLA Klasse I, die sich stark an Granulozyten, Monozyten und Lymphozyten binden
- Figur 4: Zellzahlbestimmung der weißen Blutkörperchen, Granulozyten, Monozyten und Lymphozyten vor (linke Säule) und nach der Isolation mittels Ficoll-Dichte-Gradient inklusive Zentrifugation (mittlere Säule) und Ficoll-Dichte- Gradient ohne Zentrifugation (rechte Säule)
- Figur 5: Untersuchung von 140 Seren
- Figur 6: Verdünnungsreihe der Seren, die spezifische humane IgG gegenüber HNA-1a, -1b, -2a und -3a aufweisen
- Figur 7: Intra- und Inter-Versuchs-Variationens-Bestimmungen, um die Stabilität des FLOW GIFT Versuchs, Assay, zu untersuchen und
- Figur 8: das erfindungsgemäße Prinzip des Durchfluss-zytrometrischen Granulozyten-Immunfluoreszenz-Tests (FLOW GIFT).

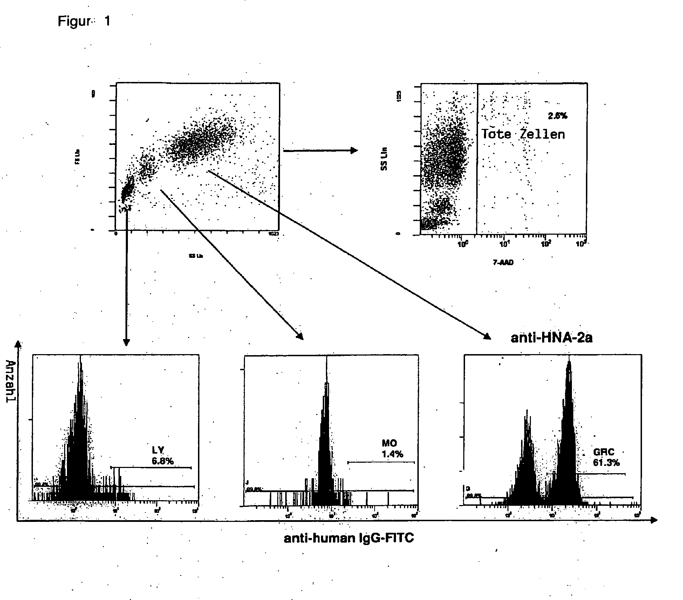

Gemäß der Figur 1 wurden Granulozyten GRC, Monozyten MO und Lymphozyten LY in Histogramm 1 differenziert, aufgezeigt im seitlichen und nach vorne gerichteten Lichtstreuung. Alle im Histogramm 1 detektierten Zellen erscheinen im Histogramm 2, gebildet vom vierten Fluoreszenzkanal sowie der seitlichen Streuung. In diesem Histogramm wurden die toten Zellen mittels 7-AAD (Emissionsspektrum von 650 nm) untersucht, um die zytotoxischen Antikörper zu analysieren. Granulozyten, Monozyten und Lymphozyten wurden einzeln in jedem Histogramm auf Zell-bindende Fluoreszenz untersucht, die sich durch den ersten Fluorenszenzkanal (FITC) sowie die Zellzahl zusammensetzt In diesem Beispiel wurde ein Serum mit Anti-HNA-2a analysiert. Die spezifischen Antikörper finden sich nur an Granulozyten gebunden. Das Histogramm zeigt ein typisches Bild von Anti-HNA-2a Antikörpern mit einem bi-phasischen Histogramm.

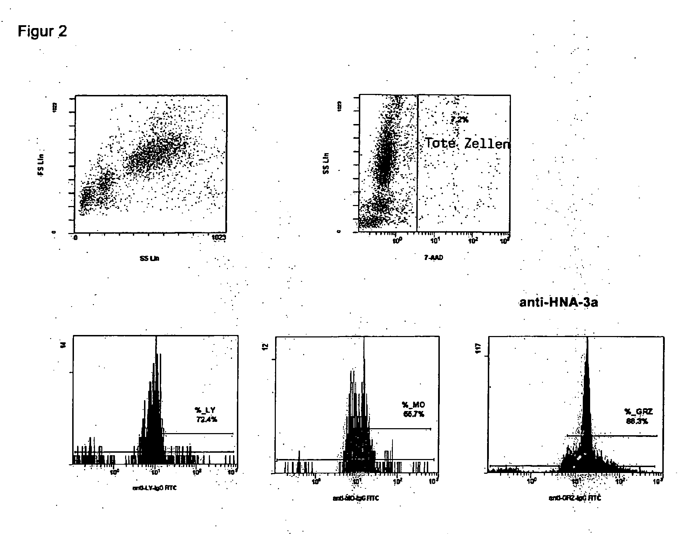

Figur 2 zeigt ein Beispiel einer durchflusszytometrischen Analyse eines Serums mit Granulozyten-spezifischem IgG Anti-HNA-3a. Dieser Antikörper bindet sich sowohl an Granulozyten als auch an Monozyten und Lymphozyten, da diese Zellpopulationen alle HNA-3a aufweisen. Der Anti-HNA-3a zeigt einen schwachen zytotoxischen Effekt (7,2% 7-AAD positive Zellen). Antikörper gegen HLA wurden mittels ELISA ausgeschlossen.

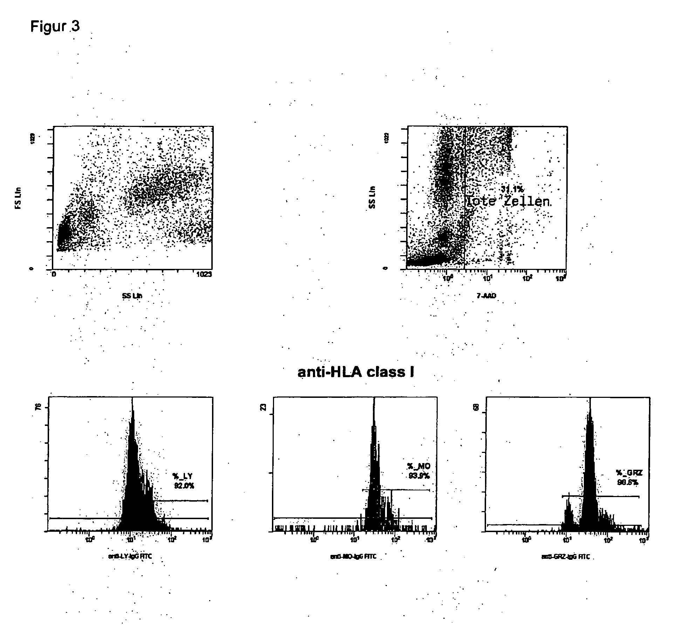

Figur 3 zeigt ein Beispiel einer durchflusszytometrischen Analyse eines Serums mit zytotoxischen und nicht-zytotoxischen Antikörpern gegen HLA Klasse I, die sich stark an Granulozyten, Monozyten und Lymphozyten binden. Die Zytotoxizität dieser Antikörper ist ursächlich für den Zelltod verantwortlich. Die toten Zellen sind 7-AAD positiv (31% 7-AAD positive Zellen).

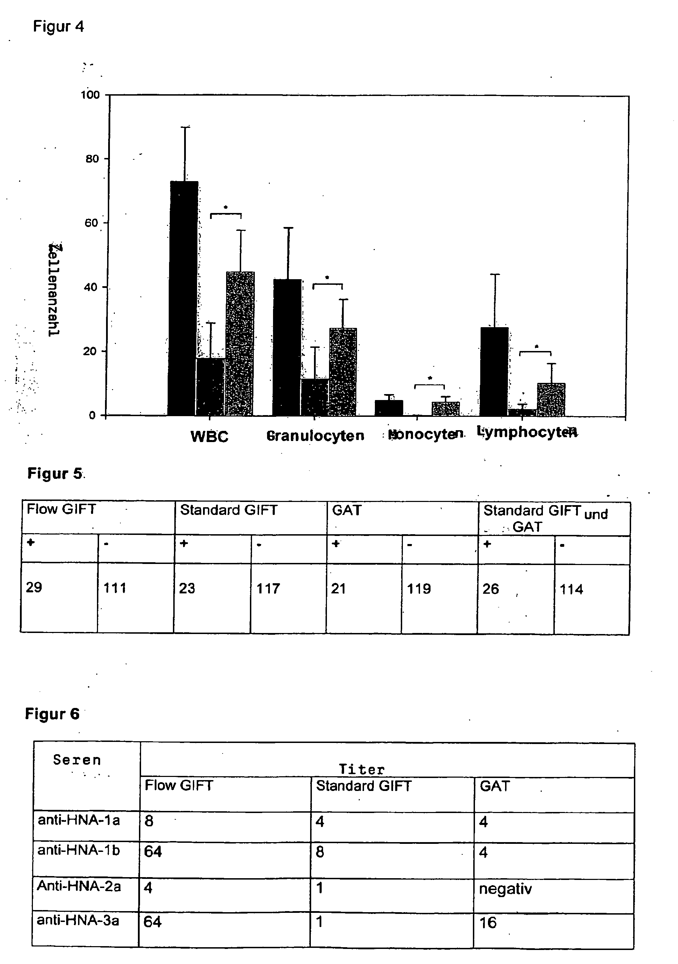

Figur 4 zeigt eine Zellzahlbestimmung, WBC, der weißen Blutkörperchen, Granulozyten, Monozyten und Lymphozyten vor (linke Säule) und nach der Isolation mittels Ficoll-Dichte-Gradient inklusive Zentrifugation (mittlere Säule) und Ficoll-Dichte- Gradient ohne Zentrifugation (rechte Säule). Für den Standard-GIFT und GAT, ist es nötig, die Granulozyten zu isolieren, indem die Monozyten und Lymphozyten entfernt werden. Hierbei konnte ein eindeutiger Verlust aller verbleibenden Zellen nach der Isolierung mittels Ficoll-Dichte-Gradient, inklusive Zentrifugation, beobachtet werden (*p< 0.002, 0.02, 0.0002 und 0.003 im gleichen Maß für weiße Blutkörperchen (WBC), Granulozyten, Monoxyten bzw. Lymphozyten).

Figur 5 zeigt eine Untersuchung von 140 Seren, die Anzahl der positiv und negativ getesteten Seren auf Bindung von Granulozyten-IgG im FLOW GIFT gegenüber dem Standard-GIFT, GAT sowie Standard-GIFT in Kombination mit GAT.

Figur 6 zeigt eine Verdünnungsreihe der Seren, die spezifische humane IgG gegenüber HNA-1a, -1b, -2a und -3a aufweisen. Die Titer im Flow GIFT, Standard-GIFT und GAT wurden entsprechend dargestellt. FLOW GIFT erwies sich hierbei gegenüber dem Standard-GIFT und GAT als empfindlicher.

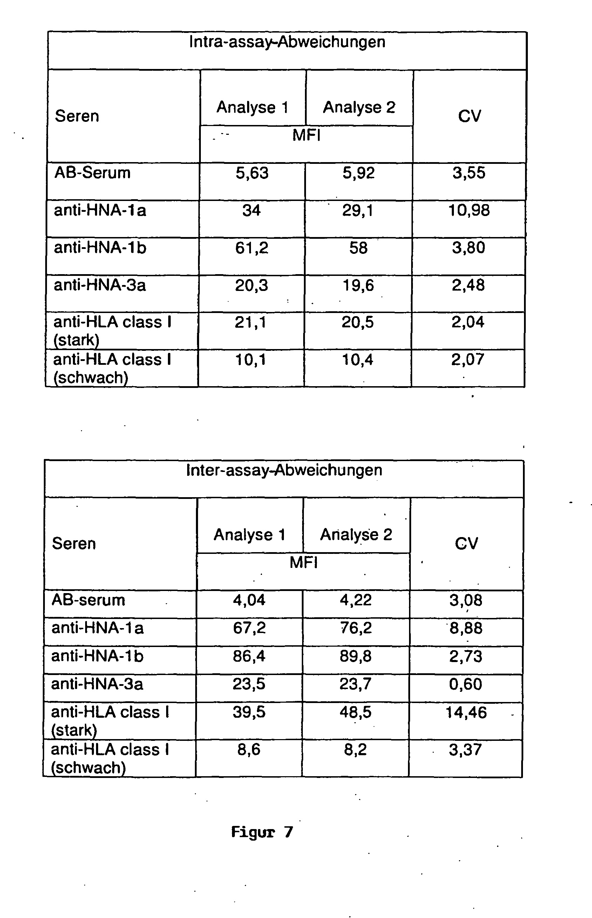

In Figur 7 wurden Intra- und Inter-Proben- (Assay-) Variationen bestimmt, um die Stabilität des FLOW GIFT Assays zu untersuchen. AB-serum, 3 Seren mit Anti-HNA1a, -1b, -3a und 2 Seren mit einem starken und einem schwachen Anti HLA Klasse I Antikörper wurden zweifach und zu verschiedenen Zeitpunkten untersucht um jeweils die Intra- und Inter-Assay-Variationen zu bestimmen. Der Koeffizient der Variation (CV) wurde von jedem einzelnen Serum berechnet.

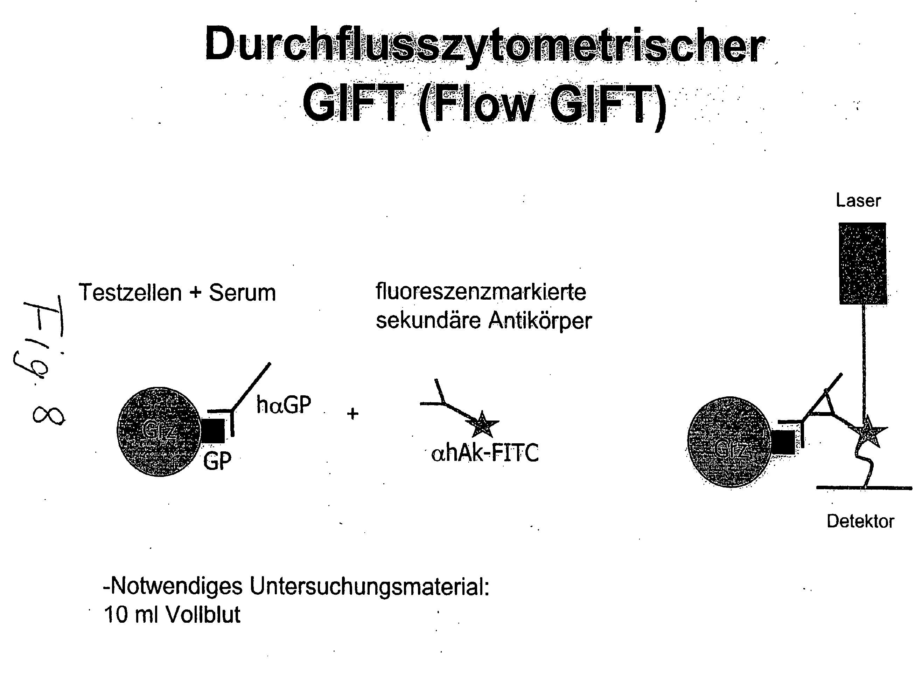

In Figur 8 ist das erfindungsgemäße Prinzip des Durchfluss-zytrometrischen Granulozyten-Immunfluoreszenz-Tests (Flow GIFT) dargestellt. Testzellen zusammen mit Serum, beispielsweise Grz-GP + hαGP, werden mittels fluoreszenzmar kierten sekundären Antikörpern markiert, beispielsweise αhAK-FITC, und anschließend wird mittels Anregung durch einen Laserdetektor das Fluoreszenzlicht der fluoreszenzmarkierten sekundären Antikörper mittels eines Detektors detektiert.

Gemäß der Erfindung ist es möglich eine genügende Anzahl von Testzellen zu isolieren für die Zurverfügungstellung von ca 150 Seren gewonnen aus 10 ml Vollblut eins gesunden Blutspenders mit einer normalen Blutzellenzählung.

Im Vergleich hierzu bei der Verwendung der bekannten Methode gemäß den Standart GIFT- und GAT-Verfahren der Laboratorien-Routine waren die isolierten Zellen nur für die Durchführung von 5 Seren ausreichend. Diese Methode gemäß der Erfindung ermöglicht auch eine rasche Durchführung, da die Zahl der Waschschritte verringert wurde und insbesondere auch auf die Fixation von Zellen verzichtet werden konnte. So konnte eine vollständige Testdurchführung in 2 Stunden erreicht werden.

Wie in der Verdünnungsreihe von Seren mit verschiedenen Granulozyten-spezifischen Antikörpern gezeigt wurde, konnte Flow GIFT, gemäß der Erfindung, Anti-HNA-1a, -1b, -2a und -3a in höheren Verdünnungsstufen erfassen als Standard-GIFT und GAT. Besonders auffällig ist, dass 3 Seren mit IgG Anti-CD16 und HLA Klasse I Antikörpern mittels FLOW GIFT erfasst wurden. Die Daten gemäß der Erfindung zeigen, dass FLOW GIFT in der Lage ist, spezifische Antikörper mit einer höheren Sensitivität zu detektieren im Vergleich zum Standard-GIFT in Kombination mit GAT. Diese Beobachtung kann damit erklärt werden, dass PFA zur Fixation von Zellen in der Standard-GIFT Methode eingesetzt wird, um eine besseren Unterscheidung von positiven und negativen Ergebnissen in der mikroskopischen Auswertung zu erreichen. Tatsächlich ist die PFA Fixation von Zellen und Gewebe als kritisch hinsichtlich der Antigenität, wie bereits bekannt, von Membran-bindenden Molekülen anzusehen. Hinsichtlich einer besseren Unterscheidung von Zellreihen mittels durchflußzytometrischer Analyse ist eine Zellfixation mit PFA nicht notwendig beim FLOW GIFT Verfahren. Ferner erwies sich die Verwendung von 7-AAD zur Erkennung von zytotoxischen Antikörpern, durch die Untersuchung von toten Zellen, sowie der simultanen Auswertung von zellgebundener Fluoreszenz bei Monozyten und Lymphozyten, in einigen Fällen als nützlich, Fig. 3. HNA-1 und 2 finden sich nur bei Granulozyten, so dass einzelne spezifische Antikörper gegen diese Antigene nur mittels durchfluBzytometrischer Analyse auf Neutrophilen entdeckt werden können, Fig.1. In Bezug auf HNA-3a, so ist dieses Antigen sowohl auf Neutrophilen als auch auf Lymphozyten zu finden. In der Studie gemäß der Erfindung konnten Anti-HNA-3a auch auf Monozyten nachgewiesen werden, Fig. 2. Dieser Antikörper steht häufig in Zusammenhang mit granulozytären Antikörpern bei TRALI. 3 Seren mit diesem Antikörper waren im Standard-GIFT Verfahren negativ. Durch die Auswertung mittels FLOW GIFT wurde ein starker Zusammenhang sowohl zwischen dem MFI der unverdünnten Proben und den entsprechenden Titern sowie zwischen den Verdünnungsreihen und der verwendeten Konzentration der einzelnen proben beobachtet. Diese Ergebnisse belegen, dass FLOW GIFT in der Lage ist, genauere Informationen zur Intensität der Antikörper hinsichtlich der großen MFI Skala jeder Zellpopulation, insbesondere bei Antikörpern mit hohem Titer, zu tiefem. Ein direkter Vergleich zwischen den Proben (Assays) zeigt vergleichbare Ergebnisse. 140 Seren wurden hierbei untersucht. Ein diskrepantes Ergebnis zwischen FLOW GIFT und Standard GIFT in Kombination mit GAT konnte nur bei 3 Seren beobachtet werden. Dieses Ergebnis zeigt, dass das FLOW GIFT Verfahren, ohne Verwendung des Agglutinatlons-Versuchs (Assay), in der Lage ist, Granulozyten-spezifische Antikörper mit einer annähernd identischen Spezifität im Vergleich zum Standard-GIfFT Verfahren in Kombination mit dem GAT Verfahren zu erkennen. Daher ist das FLOW GIFT Verfahren ideal geeignet für eine Automatisation bei der Untersuchung von Granulozyten Antikörpern bei einer großen Anzahl von Proben. Um die Stabilität des FLOW GIFT Verfahrens zu belegen, wurden Inter- und Intra-Assay-Variationen durchgeführt. Der niedrige CV Wert unterstreicht die Stabilität und Genauigkeit des FLOW GIFT Verfahrens.

Zusammengefasst bietet das FLOW GIFT Verfahren eine einfache und rasche Methode zur Bestimmung von Granulozyten-assoziierten Antikörpern wobei es alle Vorteile des Standard-GIFT Verfahrens in Kombination mit dem GAT Verfahren aufweist. Eine geringere Anzahl von Zellen werden benötigt um dieses Assay durchzuführen. Dieses neuartige System ermöglicht den Nachweis von Anti-Granulozyten-Antikörper bei einer größeren Spenderpopulation.

MATERIALIEN UND METHODEN: Insgesamt wurden 140 Seren auf das Vorhandensein von granulozytären Antikörpern untersucht. 29 Seren, bei denen zuvor die Antigene HNA-1a; -1b; -1c: -2a; -3a; -4a und -5a mittels GAT und/oder GIFT detektiert wurden, und 111 Seren mit vermutetem TRALI wurden in Verbindung gebracht. Als Testzellen wurden Vollblutproben von HNA-typisierten Spendern mittels Zellsedimentation in einem Ficoll Dichte Gradienten isoliert. Leukozyten wurden mit dem entsprechenden Serum inkubiert; die Antikörperbindung an die Testzellen wurde mittels FITC-konjugierten antihumanen Antikörpern ermittelt. Standard GIFT und GAT wurden als Referenzmethoden verwendet. ERGEBNISSE: 27 Seren waren im FLOW GIFT positive, während 6 Seren, welche anti-HNA 3a (3 x), anti-CD16 (1x) and -HLA class 1 (2x) enthielten, negativ im Standart GIFT waren. Das neue FLOW GIFT Verfahren war in der Lage alle granulozytären Antikörper zu erkennen, die sonst nur durch eine Kombination von Standard-GIFT und GAT zu bestimmen waren. Mittels GAT konnten 8 Seren mit anti-HNA 2a (2x), -CD16 (2x) und -HLA Klasse I (4x) nicht ermittelt werden. In einer fortlaufenden Verdünnungsreihe konnte das FLOW GIFT Verfahren die Antikörper in höheren Verdünnungen detektieren, als die Referenzmethoden GIFT und GAT. SCHLUSSFOLGERUNG: Das FLOW GIFT Verfahren ermöglicht eine schnelle Erkennung von granulozytären Antikörpern, wobei eine geringere Menge von Spender-Testzellen benötigt werden. Dieses Verfahren wird möglicherweise den Weg für eine Untersuchung (screening) von granulozytären Antikörpern bei großen Spenderpopulationen eröffnen.

### MATERALIEN UND METHODEN:

### Blutproben

140 eingefrorene Spenderseren, die im Zusammenhang mit vorhergegangenen Transfusionsreaktionen standen, wurden auf das Vorhandensein Granulozytenreaktiven Antikörpern untersucht. Testgranulozyten wurden aus frisch gewonnenem Na₂EDTA Vollblut von gesunden Spendern gewonnen, bei denen zuvor die neutrophilen Antigene HNA-1a; -1b; -1c; -2a: -3a; -4a und -5a typisiert wurden. Seren der AB Blutgruppe von gesunden Spendern ohne Leukozyten-spezifische Antikörper wurden als normale Kontrollen verwendet. Alle untersuchten Testseren wurden in konventionellen GAT und GIFT im Vergleich mit dem FLOW GIFT untersucht.

### Granulozyten Immunfluoreszenz- (GIFT) und Agglutinationtest (GAT):

Die Standard-Assays GIFT und GAT wurden wie beschrieben durchgeführt. Kurz gefaßt Granulozyten aus EDTA antikoaguliertem Blut von 3 gesunden Blutspendern wurden untersucht. Granulozyten wurden aus dem Überstand von leukozytenreichem Plasma mittels Ficoll-Dichte-Gradient Zentrifugation (Polymorphprep, Axis-Shield, Oslo, Norwegen) isoliert. Nach erfolgter Lysis der roten Blutkörperchen mittels PeliLyse Buffer A1 (Sanquin, Amsterdam, Niederlande), wurde die Zellsuspension zweimal in PBS, bestehend aus 0,2% BSA und 0,5% Na2-Ethylenadiaminetetraacetete (EDTA), gewaschen. Für das GIFT Verfahren wurden die Zellen mit 1% Paraformaldehyd in Mikrotiter-Pletten fixiert und erneut zweimal gewaschen. Nach einer 30 minütigen Inkubationszeit bei 37°C wurden die entsprechenden Seren hinzugefügt und erneut gewaschen (4 mal); die Zellen wurden dann mit Fluoreszein-Isothiozyanat (FITC)-markiertem Kaninchen F(ab')₂-antihumanem IgG (Dako, Hamburg, Deutschland) inkubiert, erneut 3 mal gewaschen und mittels eines Fluoreszenzmikroskops untersucht.

Der GAT-Assay wurde in Terasaki-Platten durchgeführt. (Greiner, Frickenhausen, Deutschland). Zwei Mikroliter Granulozyten, gewonnen wie zuvor beschrieben, wurden mit 2 µL Serum für 120 Minuten bei 37°C inkubiert und dann mikroskopisch ausgewertet. Die Ergebnisse wurden mit folgenden Werten berechnet 0-4 Punkteskala für das GIFT Verfahren und in Prozent (0-100%) für das GAT Verfahren.

### Differenzierung der Granulozyten-spezifischen Antikörper und Nachweis von HLA Antikörpern:

Die Differenzierung von Granulozyten-spezifischen Antikörpern mittels des GIFT und GAT Verfahrens wurde durch die monoklonale Antikörper spezifische Immobilisation von Granulozyten-Antigene Assay (MAIGA) mit MoAb durchgeführt: CD16 Klon 3G8, CD18 Klon 7E4, CD11a Klon 25,3, CD11b Klon Bear1 jeweils von Immunotech, Marseilles, Frankreich und CD177 Klon MEM 166 (Becton Dickinson, Heidelberg, Deutschland). Zur Bestimmung der HLA Antikörper wurde der Enzym-gebundene, immunosorbente Probe (Assay) (ELISA; Biotest, Dreieich, Deutschland) oder ein Flow PRA Assay von One Lambda (Canoga Park, USA) eingesetzt.

### Durchflußzytometrische Granulozyten Immunfluoreszenz (Flow GIFT):

Um Leukozyten für das erfindungsgemäße FLOW GIFT Verfahren zu isolieren, wurde 9 ml EDTA antikoaguliertes Blut von 2 gesunden Blutspendern zu 15 ml Ficoll Dichte Gradient, ohne Zentrifugationsschritt, hinzugefügt (Ficoll-Paque Plus, GE Healthcare, Uppsala, Schweden). Nach einer Wartezeit von 20 Minuten bei Raumtemperatur, werden die Leukozyten automatisch mittels Sedimentation vom leukozytenreichen Plasmaüberstand getrennt. Die im Überstand verbleibenden roten Blutkörperchen (RBCs) wurden durch Zugabe von 10%tigem Ammoniumchloride lysiert. Die Zellen wurden anschließend in PBS, bestehend aus 0,2% BSA und 0,5% Na₂-EDTA, gewaschen und in 7 ml PBS resuspendiert. 50µl der resuspendierten Zellen wurden mit 50 µl des entsprechenden Serums für 30 min bei 37°C inkubiert; danach folgten 2 Waschschritte sowie eine Inkubation mit FITC-markiertem Kaninchen F(ab')₂-anti-humanes IgG (Dako, Hamburg, Deutschland). Um tote Zellen auszuschließen, wurde 7-AAD (Beckman Coulter, Krefeld, Deutschland) hinzugefügt. Nach einer 30 minütigen Inkubationszeit bei Raumtemperatur, folgte ein Waschschritt sowie eine Resuspension in 300 µl PBS, welche 0.1%tigen Paraformaldehyd (PFA) enthält; die Zell-bindende Fluoreszenz wurde mittels einer durchflußzytometrischen Analyse bestimmt.

Alle durchflußzytometrischen Analysen wurden mittels der FC 500 Maschine (Beckman-Coulter, Krefeld, Deutschland) durchgeführt. Die Einstellungen am Durchflußzytometer wurden auf die lineare Verstärkung der Lichtstreuung und die logarithmische Verstärkung von 5 Fluoreszenz Kanälen justiert. Die Grenzwerte wurden in einer nach vorne gerichteten Streuung definiert um Zellüberreste auszuschließen.

Granulozyten, Monozyten und Lymphozyten wurden als seitliche und nach vorne gerichtete Streuung aufgezeigt. Nach der back gating im vierten FL Kanal gegenüber der seitlichen Streuung wurden die toten Zellen (7-AAD-positive Zellen) analysiert. Granulozyten, Monozyten und Lymphozyten wurden getrennt auf xell-bindende Fluoreszenz analysiert (Fig. 1 und 2). Mindestens 5000 Events pro Zellpopulation wurden gemessen. Die durchschnittliche Fluoreszenzintensität (MFI) wurde als Wert benannt. Der cut-off Wert wurde als Verhältnis von MFI zu Testserum und AB-Serum definiert. Das Serum wurde als positiv bewertet, wenn der Verhältniswert > 2 betrug.

### Analyse der restlichen Zellen nach der Zellisolation aus Vollblut:

Um die verbleibenden Zellen nach der Ficoll Dichte Gradient Isolation mit oder ohne Zentriflugationsschritt zu analysieren, wurden 10 ml EDTA-Vollblutproben von 10 gesunden Blutspendern anhand beider Isolationsmethoden entsprechend vorbereitet. Das Differenzialblutbild von Vor und Nach der Zellisolation wurde mittels des Blutbildautomats Cell-Dyn 3700 (Abbott, Wiesbaden-Delkenheim, Deutschland) ausgezählt. Die Gesamtzahl der weißen Blutkörperchen (WBC), Granulozyten, Monozyten und Lymphozyten, wurde analysiert.

### Titration von Seren mit spezifischen Granulozyten bindenden Antikörpern und Stabilitätstestung des Flow GIFT:

Seren mit Antikörpern gegen HNA-1a, -1b, -2a und -3a wurden in Serie mit AB-Serum verdünnt. Um die Stabilität des FLOW GIFT zu untersuchen, wurden sowohl eine Inter- als auch eine Intra-Assay-Abweichung durchgeführt. Das AB-Serum und 5 weitere Seren mit Anti-HNA1a, -1b, -3a und HLA Klasse I (2 Seren mit je einem starken und einem schwachen Antikörper) Antikörpern wurden zweifach analysiert um die Intra-Assay-Abweichung zu bestimmen. Für die Inter-Assay-Abweichung wurden die gleichen Seren zu unterschiedlichen Zeitpunkten untersucht.

### Statistische Analyse und Berechnung:

Durchschnittswerte, Standardabweichungen und Koeffizienten der Abweichungen wurden entsprechend berechnet. Die Unterschiede bei den Angaben gelten als statistisch signifikant bei einem Wert von p< 0,05. Alle Tests wurden mit Hilfe von handelsüblicher Software für Personal Computer durchgeführt (SPSS für Windows NT, SPSS Software GmbH, München, Deutschland).

### ERGEBNISSE:

### Gewinnung von Granulozyten, Monozyten und Lymphozyten nach der Zellisolation mittels Ficoll Dichte Gradient mit oder ohne Zentrifugation:

Die Gesamtzahl der verbleibenden Zellen im Anschluss an die Ficoll Dichte Gradient Isolation, mit oder ohne Zentrifugationsschritt, wurde analysiert. Vor der Zellisolation lag die Gesamtzahl der weißen Blutkörperchen bei 73 ± 16.9 (55-100) x 10⁶ mit 43 ± 17 (25-72) x 10⁸ Granulozyten, 5 ± 2 (2,6-8,5) x 10⁶ Monozyten und 27,7 ± 16,6 (16,2-72,8) x 10⁸ Lymphozyten. Nach der Zellisolation mittels Ficoll Dichte Gradient, mit oder ohne Zentrifugationsschritt, lag die Zahl der weißen Blutkörperchen bei 18 ± 11 (1,33 - 42,1) x 10⁶ vs. 45 ± 13 (30-63) x 10⁸ mit 11.5 ± 10 (1,1-36,3) x 10⁸ vs. 27,5 ± 9 (16-43) x 10⁶ Granulozyten, 0,22 ± 0,1 (0,1-0,45) x 10⁶ vs. 4,4 ± 1,7 (2,1~7,74) x 10⁶ Monozyten und 2,22 ± 1,56 (0,14-5,81) x 10⁸ vs. 10,3 ± 6,1 (2,1-19,6) x 10⁸ Lymphozyten. Demnach konnte nach einer Isolation mit Ficoll-Dichte-Gradient und einem Zentrifugationsschritt ein beträchtlicher Verlust an Zellen der einzelnen verbliebenen Zellpopulationen beobachtet werden (p< 0,02) (Fig. 4).

### Spezifität:

Um die Spezifität von FLOW GIFT im Vergleich zum Standard-GIFT und GAT Verfahren zu bestimmen, wurden 140 Seren von Spendern, die in Verbindung mit Transfusionsreaktionen gebracht wurden, oder von Patienten mit verschiedenen Grunderkrankungen, untersucht. Von den 140 Seren wurden 29 Seren, welche Granulozyten-spezifische Antikörper gegen Anti-HNA-1a (4x), -1b (4x), -1c (1x); - 2a (3x); -3a (11x); -CD16 (2x) und HLA-Klasse I (4x) enthielten, in einem Ringversuch untersucht und mittels FLOW GIFT bestätigt. Die Spezifität der Antikörper wurde im Rahmen einer detaillierten Untersuchung typisierten Granulozyten, mittels MAIGA und HLA-ELISA bestätigt. Von 29 im FLOW GIFT positiv getesteten Seren, wurden 23 Seren mit dem Standard-GIFT Verfahren erkannt. 111 Seren waren in beiden Assays negativ (Figur 5). 6 Seren enthielten Anti-HNA 3a (n= 4), Anti-CD16 (n= 1) und -HLA Klasse 1 (n= 2) und waren lediglich positiv im Flow GIFT, jedoch negativ im Standard-GIFT Verfahren. Bezüglich der Ergebnisse des GAT Verfahrens, waren 21 Seren positiv und 119 Seren negativ. 8 Seren enthielten Anti-HNA 2a (n= 2), Anti-Cd 6 (n= 2) und -HLA Klasse I (n= 4) und wurden nicht im GAT erkannt. Von den 29 im FLOW GIFT positiv getesteten Seren waren 3 Seren negativ im Standard-GIfT Verfahren und auch im GAT Verfahren (1x Anti-CD16 und 2 x HLA-Klasse I). 4 Seren enthielten Anti-HNA-3a und wurden nur im GAT Verfahren erkannt. Bei der durchflusszytometrischen Analyse waren die Antikörper bezüglich HLA Klasse I und 4 Proben mit Anti-HNA-3a positiv bei den Populationen von Lymphozyten und Monozyten.

### Sensitivität und Stabilität des FLOW GIFT Verfahrens:

Um die Sensitivität des FLOW GIFT Verfahrens zu bestimmen, wurden 4 Seren, jeweils mit bekannten spezifischen Granulozyten bindenden Antikörpern (Anti-HNA-1a, -1b, -2a, -3a), mit AB-Serum verdünnt und im FLOW GIFT sowie Standard-GIFT und GAT untersucht. Figur 6 zeigt die Verdünnungsreihe der Seren. Alle gemessenen Werte der Verdünnungsreihe steigen im Verhältnis zur verwendeten Konzentration proportional ab. Bei allen Seren konnte das FLOW GIFT Verfahren die Granulozyten-spezifischen Antikörper in einer höheren Verdünnung als das Standard-GIFT und/oder GAT Verfahren detektieren. Eine starke Übereinstimmung konnte sowohl zwischen dem MFI der unverdünnten Proben und den entsprechenden Titem als auch zwischen der Verdünnungsreihe und der jeweiligen Konzentration, die für die einzelnen Seren verwendet wurden, beobachtet werden (p< 0.01).

Um die Stabilität des FLOW GIFT Verfahrens zu belegen, wurden Intra- und Inter-Assay-Variationen durchgeführt. Das AB-Serum sowie 5 weitere Seren mit Anti-HNA1a, -1b, -3a und HLA Klasse I (2 Seren wiesen je einen starken und einen schwachen Antikörper auf) wurden zweifach untersucht um die Intra-Assay Abweichung zu bestimmen. Die durchschnittliche Standardabweichung (Koeffrizient der Abweichung) der einzelnen Seren wurde berechnet. Der Abweichungskoeffizient der Intra-Assay-Variation lag bei 4,5 ± 3,43% (2,04-10,98). Für die Inter-Assay Variation wurden die gleichen Seren untersucht, jedoch zu unterschiedlichen Zeitpunkten. Der Koeffizient der Variation wurde für jedes Serum berechnet und ergab 5,5 ± 5,2 (0,6-14,46) % (Figur 7).

Die Durchflusszytometrie gemäß der Erfindung zeigte sich als sensible Verfahren zur Detektion von Leukozyten-bindenden Immunglobulinen. Die Durchflusszytometrie ermöglicht die Unterscheidung verschiedener Analyte bei einer einzelnen Probe im Hinblick auf Größe und Farbe. Die bislang verwendeten Methoden zur Isolation von Zellen führten jeweils zu einem hohen Zellverlust. Deshalb wurde eine große Menge Vollblut von gesunden Blutspendern für die Isolation von Testzellen benötigt, um eine große Anzahl von Blutproben zu untersuchen. Unter diesem Aspekt ist es gelungen, ein Verfahren zu etablieren, mit dem ein rascher Nachweis von Granulozyten Antikörpern durchgeführt werden kann und die Zahl der dabei benötigten Zellen möglichst gering gehalten wird. Darüber hinnaus ist es gelungen, weitere Informationen über die Antikörperbindung an weiteren Zellreihen wie den Monozyten und Lymphozyten zu erhalten sowie über das Zellsterben aufgrund möglicher komplementärer aktivierender Antikörper.

Um einen hohen Verlust von Zellen zu vermeiden, ist das aktuelle Zellisolationsverfahren nach dem Stand der Technik erfindungsgemäß so modifiziert worden, dass es das Ficoll-Dichte-Gradient Verfahren ohne Zentrifugation verwendet. Jede Zellpopulation wird mittels Sedimentation getrennt. Mit dieser Methode können Granulozyten, Monozyten und Lymphozyten in einer ausreichenden Menge gewonnen werden, um eine Untersuchung einer großen Anzahl von Blutproben durchzuführen. Auf diese Weise ist es möglich, eine genügende Anzahl von Testzellen für die Durchführung von einem Minimum von 150 Seren aus 10ml Vollblut von einem gesunden Blutspender mit einem normalen Blutzellen-Zähler zu gewinnen. Im Vergleich bei der Verwendung der bekannten Methode gemäß den Standart GIFT- und GAT-Verfahren der Laboratorien-Routine sind die isolierten Zellen nur für die Durchführung von 5 Seren ausreichend. Diese Methode ermöglicht auch eine rasche Durchführung, da die Zahl der Waschschritte verringert ist und insbesondere auch auf die Fixation von Zellen verzichtet wird. So kann eine vollständige Testdurchführung in 2 Stunden erfolgen.

Wie in der Verdünnungsreihe von Seren mit verschiedenen Granulozyten-spezifischen Antikörpern gezeigt ist, kann der FLOW GIFT Anti-HNA-1a, -1b, -2a und -3a in höheren Verdünnungsstufen erfassen als Standard-GIFT und GAT. Besonders auffällig ist, dass 3 Seren mit IgG Anti-CD16 und HLA Klasse I Antikörpern mittels des FLOW GIFT erfasst werden. Die vorgestellten Daten zeigen, dass der FLOW GIFT in der Lage ist, spezifische Antikörper mit einer höheren Sensitivität zu detektieren im Vergleich zum Standard-GIFT in Kombination mit GAT. Diese Beobachtung kann damit erklärt werden, dass PFA zur Fixation von Zellen in der Standard-GIFT Methode eingesetzt wird, um eine besseren Unterscheidung von positiven und negativen Ergebnissen in der mikroskopischen Auswertung zu erreichen. Tatsächlich ist die PFA Fixation von Zellen und Gewebe als kritisch hinsichtlich der Antigenität von Membran-bindenden Molekülen anzusehen. Zur besseren Unterscheidung von Zellreihen mittels durchflußzytometrischer Analyse ist eine Zellfixation mit PFA beim FLOW GIFT Verfahren nicht notwendig. Ferner erwies sich die Verwendung von 7-AAD zur Erkennung von zytotoxischen Antikörpern, durch die Untersuchung von toten Zellen, sowie der simultanen Auswertung von zellgebundener Fluoreszenz bei Monozyten und Lymphozyten, in einigen Fällen als nützlich (Fig. 3). HNA-1 und 2 finden sich nur bei Granulozyten, so dass einzelne spezifische Antikörper gegen diese Antigene nur mittels durchflußzytometrischer Analyse auf Neutrophilen entdeckt werden können (Fig.1). In Bezug auf HNA-3a, so ist dieses Antigen sowohl auf Neutrophilen als auch auf Lymphozyten zu finden. In der vorgestellten Studie konnten Anti-HNA-3a auch auf Monozyten nachweisen werden (Fig. 2). Dieser Antikörper steht häufig in Zusammenhang mit granulozytären Antikörpern bei TRALI. 3 Seren mit diesem Antikörper waren im Standard-GIFT Verfahren negativ. Durch die Auswertung mittels FLOW GIFT kann ein starker Zusammenhang sowohl zwischen dem MFI der unverdünnten Proben und den entsprechenden Titern sowie zwischen den Verdünnungsreihen und der verwendeten Konzentration der einzelnen Proben hergeleitet werden. Diese Ergebnisse belegen, dass der FLOW GIFT in der Lage ist, genauere Informationen zur Intensität des Antikörpers hinsichtlich des großen MFI Skalas der Zellpopulationen, insbesondere bei Antikörpern mit hohem Titer, zu liefern. Diese Ergebnisse belegen, dass der FLOW GIFT in der Lage ist, genauere Informationen zur Intensität des Antikörpers hinsichtlich des großen MFI Skalas der Zellpopulationen, insbesondere bei Antikörpern mit hohem Titer, zu liefern.

Ein direkter Vergleich zwischen den Proben (Assays) zeigt vergleichbare Ergebnisse. 140 Seren wurden hierbei untersucht Ein diskrepantes Ergebnis zwischen dem FLOW GIFT und Standard GIFT in Kombination mit GAT konnte nur bei 3 Seren beobachtet werden. Dieses Ergebnis zeigt, dass das erfindungsgemäße FLOW GIFT Verfahren, ohne die Verwendung des Agglutinations-Assays, in der Lage ist, Granulozyten-spezifische Antikörper mit einer annähernd identischen Spezifität im Vergleich zum Standard-GIFT Verfahren in Kombination mit dem GAT Verfahren zu erkennen. Daher ist das FLOW GIFT Verfahren ideal geeignet für eine Automatisation bei der Untersuchung von Granulozyten Antikörpern bei einer großen Anzahl von Proben. Um die Stabilität des FLOW GIFT Verfahrens zu belegen, wurden Inter-und Intra-Assay-Variationen durchgeführt. Der niedrige CV-Wert unterstreicht die Stabilität und Genauigkeit des FLOW GIFT Verfahrens.

Zusammengefasst bietet das FLOW GIFT Verfahren eine einfache und rasche Methode zur Bestimmung von Granulozyten-assoziierten Antikörpern wobei es alle Vorteile des Standard-GIFT Verfahrens in Kombination mit dem GAT Verfahren aufweist. Eine geringere Anzahl von Zellen werden benötigt um dieses Assay durchzuführen. Dieses neuartige System ermöglicht den Nachweis von Anti-Granulozyten-Antikörper bei einer größeren Spenderpopulation.

Die Erfindung ist für das Selektieren, Screening, von Blut und/oder Serum auf granulozytäre Antikörper bei umfangreicher Spenderpopulation gewerblich anwendbar. Der besondere Vorteil besteht darin, dass durch die Erfindung eine einfache, schnelle und sensitive Methode bei geringem Verbrauch von Testzellen zur Verfügung gestellt wird. Konnte man mit der bisherigen Methode des Standes Technik mit 10 ml EDTA-Blut 6 bis 8 Untersuchungen durchführen, so erlaubt die Erfindung mit 10 ml EDTA-Blut cirka 150 Untersuchungen.

## Patentansprüche

1. Verfahren zum Selektieren, Screening, von Blut und/oder Serum auf granulozytäre Antikörper bei umfangreicher Spenderpopulation, **dadurch gekennzeichnet, dass** eine Durrhflusszytometrie mit Granulozyten-Immunfluoreszenz angewendet wird, wobei EDTA-Blut von 2 -HNA-typisierten Spendern verwendet wird und eine Zellisolierung über den Dichtegradient mittels Vollblutlyse erfolgt unter Ausschluss einer Zentrifugation und anschließend ein Waschschritt erfolgt, wonach zur Durchführung des Granulozyten-Immunfluoreszenz-Tests, eine Inkubation des isolierten Zellmaterials mittels Serum sowie eine Inkubation mit fluoreszenz-markierten Antikörpern erfolgt und das inkubierte Zellmaterial einer Durchfluss-zytometrischen Analyse unterworfen wird.

2. Verfahren nach Anspruch 1, **dadurch gekennzeichnet,**
**dass** jede Zellpopulation bei der Durchführung zur Zellisolierung durch Sedimentation mittels eines Ficoll-Dichtegradienten getrennt wird.

3. Verfahren nach Anspruch 1 oder 2, **dadurch gekennzeichnet,**
**dass** die Sedimentation durch Rütteln unterstützt wird.

4. Verfahren nach Anspruch 1, 2 oder 3, **dadurch gekennzeichnet,**
**dass** auf eine Fixation von Zellen verzichtet wird.

5. Verfahren nach Anspruch 1, 2, 3 oder 4, **gekennzeichnet durch**,
Verwendung von 7-AAD zur Erkennung von zytotoxischen Antikörpern, **durch** die Untersuchung von toten Zellen, sowie der simultanen Auswertung von zellgebundener Fluoreszenz bei Monozyten und Lymphozyten.

6. Verfahren nach einem der vorstehenden Ansprüche, **dadurch gekennzeichnet,**
**dass** der Durchtluss-Granulozyten-Imrnunfluoreszenz-Test (Flow GIFT) in einem automatisierten Verfahren mittels eines Automaten erfolgt.

7. Verfahren nach einem der vorstehenden Ansprüche, **dadurch gekennzeichnet,**
**dass** die Testzellen zusammen mit dem Serum mittels fluoreszenzmanerten sekundären Antikörpern markiert und anschließend mittels eines Laser-Detektors durch Anregung das Fluoreszenzlicht der fluoreszenzmarkierten sekundären Antikörper mittels eines Detektors detektiert wird.

8. Verfahren nach einem der vorstehenden Ansprüche, **dadurch gekennzeichnet,**
**dass**, um Leukozyten zu isolieren, antikoaguliertes Blut zu Ficoll Dichte Gradient, ohne Zentrifugationsschritt hinzugefügt wird,
anschließend nach einer Wartezeit von 10 bis 30, vorzugsweise ca. 20 Minuten, bei Raumtemperatur, eine Trennung der Leukozyten selbsttätig mittels Sedimentation vom leukozytenreichen Plasmaüberstand erfolgt,
dann im Überstand verbleibende roten Blutkörperchen (RBCs) lysiert werden, vorzugsweise durch Zugabe von 10%tigem Ammoniumchloride,
die Zeilen anschließend gewaschen werden, vorzugsweise in PBS, bestehend aus 0,2% BSA und 0,6% Na₂-EDTA,
und resuspendiert werden, vorzugsweise in PBS,
dann eine Inkubation der resuspendierten Zellen mit entsprechendem Serum erfolgt, vorzugsweise für ca. 30 min bei ca. 37°C;
danach gegebenenfalls ein Waschschritt, insbesondere 2 Waschschritte folgen,
und eine Inkubation mit FITC-markiertem, vorzugsweise mit Kaninchen F(ab')₂-anti-humanes IgG erfolgt,
wobei um tote Zellen auszuschließen, 7-AAD hinzugefügt wird,
und eine, vorzugsweise ca 30 minütige. Inkubationsteit bei Raumtemperatur abgewartet wird,
worauf gegebenenfalls ein weiterer Waschschritt sowie gegebenenfalls eine Resuspension, vorzugsweise in PBS erfolgt, welche vorzugsweise ca. 0.1%teigen Paraformaldehyd (PFA) enthält;
schließlich die zellgebundene Fluoreszenz mittels einer durchflußzytometrischen Analyse bestimmt wird.

## Claims

1. A process for selecting, screening of blood and/or serum for granulocytic antibodies in an extensive donor population, **characterized by** the application of granulocyte immunofluorescence flow cytometry, wherein EDTA blood of 2 -HNA-typed donors is used and cells are isolated by whole-blood lysis on the basis of a density gradient, without centrifugation, followed by a washing step, after which the isolated cell material is serum-incubated and incubated with fluorescence-labeled antibodies, and the incubated cell material is subjected to a flow cytometric assay, to perform the granulocytic immunofluorescence test.

2. A process according to claim 1, **characterized in that**
each cell population is separated by sedimentation using a Ficoll density gradient in order to isolate the cells.

3. A process according to claim 1 or 2, **characterized in that**
vibration is used to promote the sedimentation.

4. A process according to any one of claims 1, 2 and 3, **characterized in that** the cells are not fixated.

5. A process according to any one of claims 1, 2, 3 and 4, **characterized by**
the use of 7-AAD for detecting cytotoxic antibodies, examining dead cells, and assessing simultaneously cell-associated fluorescence in monocytes and lymphocytes.

6. A process according to any one the preceding claims, **characterized in that** the flow granulocytic immunofluorescence test (Flow GIFT) is performed in an automated process by an automatic device.

7. A process according to any one of the preceding claims, **characterized by** labeling the cells to be tested as well as the serum by means of fluorescence-labeled secondary antibodies and subsequently detecting, upon excitation, a fluorescence light of the fluorescence-labeled secondary antibodies by means of a detector.

8. A process according to any one of the preceding claims, **characterized by** adding anticoagulated blood to Ficoll density gradient, without a centrifugation step, in order to isolate leucocytes,
subsequently allowing the leucocytes to separate by self-sedimentation of supernatant leucocyte-rich plasma after a latency of 10 to 30, preferably 20 minutes, at room temperature, lyzing remaining red blood cells (RBCs), preferably by adding ammonium chlorides (10 %), subsequently washing the cells, preferably in PBS comprising BSA (0.2 %) and Na₂-EDTA (0.5 %),
and resuspending them, preferably in PBS,
incubating the resuspended cells with an appropriate serum, preferably for about 30 min at about 37 °C;
if appropriate, performing a subsequent washing step, in particular 2 washing steps,
and incubating with FITC-labeled, preferably with rabbit F(a')₂-anti-human IgG,
wherein 7-AAD is added to exclude dead cells,
and observing an incubation of preferably 30 minutes at room temperature,
followed by another washing step, if appropriate, and a resuspension, if appropriate, preferably in PBS which preferably includes paraformaldehyde (PFA) (0.1 %);
and finally determining the cell-associated fluorescence by a flow cytometric assay.

## Revendications

1. Procédé pour sélectionner et cribler du sang et/ou du sérum pour des anticorps granulocytaires dans une vaste population de donneurs, **caractérisé en ce qu'**on a recours à la cytométrie en flux avec immunofluorescence des granulocytes en utilisant du sang EDTA de 2 donneurs typés HNA et en isolant les cellules par gradient de densité à l'aide d'une lyse de sang total sans aucune centrifugation et en procédant ensuite à une étape de lavage, après quoi, pour effectuer le test d'immunofluorescence sur granulocytes, on pocède à une incubation du matériel cellulaire isolé avec du sérum ainsi qu'à une incubation avec des anticorps marqués par fluorescence, et on soumet le matériel cellulaire à une analyse cytométrique de flux.

2. Procédé selon la revendication 1, **caractérisé en ce que**
chaque population de cellules est séparée aux fins de l'isolement cellulaire par sédimentation à l'aide d'un gradient de densité Ficoll.

3. Procédé selon la revendication 1 ou 2, **caractérisé en ce que**
la sédimentation est favorisée par vibration.

4. Procédé selon la revendication 1, 2 ou 3, **caractérisé en ce qu'**
on renonce à une fixation de cellules.

5. Procédé selon la revendication 1, 2, 3 ou 4, **caractérisé par**
l'utilisation de 7-AAD pour l'identification d'anticorps cytotoxiques, par l'examen de cellules mortes ainsi que par l'analyse simultanée de la fluorescence cellulaire chez les monocytes et les lymphocytes.

6. Procédé selon l'une des revendications précédentes, **caractérisé en ce que**
le test d'immunofluorescence sur granulocytes par cytométrie en flux (Flow GIFT) s'effectue selon un procédé automatisé à l'aide d'un automate.

7. Procédé selon l'une des revendications précédentes, **caractérisé en ce que**
les cellules à tester et le sérum sont marqués par des anticorps secondaires marqués par fluorescence et que, ensuite, la lumière fluorescente des anticorps secondaires marqués par fluorescence est détectée par excitation à l'aide d'un détecteur laser.

8. Procédé selon l'une des revendications précédentes, **caractérisé en que**,
pour isoler des leucocytes, on ajoute du sang anticoagulé au gradient de densité Ficoll sans aucune étape de centrifugation,
qu'ensuite, après un temps d'attente compris entre 10 et 30 minutes, de préférence d'env. 20 minutes à la température ambiante, les leucocytes se séparent, par sédimentation, du surnageant plasmatique riche en leucocytes,
que les globules rouges (RBC) restant dans le surnageant sont lysés, de préférence par addition de chlorure d'ammonium à 10%,
que les cellules sont ensuite lavées, de préférence dans du PBS contenant 0,2% de BSA et 0,5% de Na₂-EDTA,
et resuspendues, de préférence dans du PBS,
qu'on procède alors à une incubation des cellules resuspendues avec le sérum correspondant, de préférence pendant env. 30 minutes à env. 37°C, suivie, le cas échéant, d'une étape de lavage, en particulier de 2 étapes de lavage,
et d'une incubation avec de l'IgG marqué FITC, de préférence du Lapin anti-Humain IgG F(ab')₂,
en ajoutant de la 7-AAD pour exclure les cellules mortes,
et en attendant le temps d'incubation, de préférence env. 30 minutes, à la température ambiante,
suivi, le cas échéant, d'une étape de lavage et, le cas échéant, d'une resuspension, de préférence dans du PBS, contenant de préféence du paraformaldéhyde (PFA) à env. 0, 1 %,
et que, finalement, on détermine la fluorescence cellulaire à l'aide d'une analyse cytométrique de flux.
